# DEMANDE DE BREVET EUROPEEN

(11) **EP 1 902 687 A1**
(43) Date de publication de la demande: **26.03.2008**
(21) Numéro de dépôt: 07356115.1
(22) Date de dépôt: 17.08.2007
(51) Int. Cl.: A61F 2/00

(54) **Ensemble chirurgical de traitement de l'incontinence urinaire chez l'homme**

(30) Priorité: 21.09.2006 FR 0608275
(71) Demandeur: CL MEDICAL, 69110 Sainte Foy Les Lyon (FR)
(72) Inventeur: Goria, Vincent Jean-Claude, 69005 Lyon (FR)
(74) Mandataire: Grand, Guillaume

(57) **Abrégé**

Cet ensemble chirurgical comporte au moins une bandelette allongée de soutien de l'urètre (116) d'un patient, comprenant, au moins dans sa partie longitudinale médiane (21C) destinée à s'étendre transversalement à l'aplomb de l'urètre lorsque la bandelette est implantée, un tricot de fils qui forment entre eux des espaces libres, ce tricot comprenant une armature constituée de chaînettes longitudinales (23) et un treillis intermédiaire (24) reliant transversalement ces chaînettes. Pour garantir un soutien obstructif fiable de l'urètre, l'ensemble de traitement comprend en outre des moyens de suture adaptés pour être accrochés aux chaînettes du tricot en étant rapportés à travers les espaces libres, de manière à joindre la partie médiane de la bandelette avec des tissus anatomiques sous-urétraux (118) en au moins deux zones ponctuelles d'accrochage (32 à 35) alignées suivant une direction sensiblement perpendiculaire à la direction longitudinale (27) de la bandelette.

## Description

La présente invention concerne un ensemble chirurgical de traitement de l'incontinence urinaire chez un homme, une utilisation d'un tel ensemble et une méthode chirurgicale de traitement de l'incontinence urinaire chez un homme, au moyen de cet ensemble. Elle a également trait à une bandelette de soutien de l'urètre d'un homme pour traiter son incontinence.

L'incontinence urinaire chez l'homme après prostatectomie est une affection invalidante qui altère la qualité de la vie même pour une incontinence modérée. Le nombre de patients incontinents a augmenté ces dernières années avec l'avènement des possibilités de dépistage précoce du cancer de la prostate et la réalisation d'un nombre plus élevé de prostatectomies totales. Même si les techniques opératoires récentes permettent de mieux préserver le sphincter, des fuites d'urine peuvent survenir dans des pourcentages de cas non négligeables : ces fuites d'urine sont liées à la technique chirurgicale qui fait sectionner la partie basse de la prostate juste au voisinage du sphincter, mais peuvent également être favorisées par des éléments constitutifs liés au malade, comme une longueur d'urètre courte, un sphincter faible ou fatigable, etc.

Pour les patients souffrant d'une incontinence urinaire malgré une rééduction de leur sphincter, le traitement chirurgical couramment employé ces dernières années consiste à implanter un sphincter artificiel. Cette intervention limite significativement l'incontinence mais souffre d'inconvénients majeurs : le patient doit manipuler une pompe à chaque miction, le matériel implanté est onéreux et le taux de réintervention est élevé.

Une nouvelle technique chirurgicale de traitement est donc apparue ces toutes dernières années, à savoir la pose d'un implant de soutien sous-urétral, se présentant généralement à la façon d'un filet sous forme d'une plaque. Ce genre de filet nécessite d'être fermement amarré aux structures anatomiques du patient, pour garantir un soutien efficace de l'urètre et corriger ainsi l'incontinence. La plaque est ainsi soit suspendue par des fils remontant de chaque côté de la vessie par voie sus- ou rétro-pubienne, avec fixations terminales dans la paroi abdominale du patient, soit directement accrochée par des vis dans les branches osseuses ischiopubiennes du patient. Dans le premier cas, les risques de perforation vésicale sont réels et, dans le second cas, l'intervention chirurgicale, plus complexe, expose le patient à un risque d'ostéite. Dans les deux cas, la présence de la plaque implantée peut entraîner un inconfort périnéal transitoire postopératoire, voire des douleurs périnéales permanentes. De plus, même si à court terme, le taux de succès du traitement est élevé, il tend à baisser significativement au cours du temps.

Dans le même temps, des matériels et des méthodes chirurgicales de traitement de l'incontinence chez la femme ont été développés avec succès ces dernières années. En particulier, on connaît, par EP-A-1 342 450 au nom de la présente Demanderesse, une bandelette de soutien sous-urétrale, pouvant être implantée, entre autres, par une voie dite « transobturatrice », c'est-à-dire par passage des parties d'extrémité libre de la bandelette à travers respectivement les trous obturateurs de l'os iliaque d'une patiente, créant ainsi un effet de suspension pour l'urètre au niveau de la partie longitudinale médiane de la bandelette. Les intérêts de cette voie d'implantation transobturatrice sont réels, puisqu'elle ne fait courir aucun risque de perforation vésicale et ne nécessite aucune fixation osseuse, seules les extrémités libres de la bandelette étant amarrées dans l'abdomen de la patiente.

Egalement dans le domaine du traitement de l'incontinence chez la femme, FR-A-2 859 624 et US-A-2002/0138025 proposent des bandelettes tricotées dont la partie médiane forme un soutien souple de l'urètre, tandis que les extrémités longitudinales de la bandelette sont à ancrer fermement dans le corps de la patiente, à distance de son urètre, en particulier au moyen de sutures. Les bandelettes proposées dans ces deux documents donnent des résultats satisfaisants chez la femme, mais sont inutilisables, voire contre-indiquées chez l'homme. En effet, la bandelette de FR-A-2 859 624 est tricotée de telle sorte que sa partie médiane est significativement plus élastique que ses extrémités dont la densité de tricotage est nettement plus forte pour rendre ces extrémités résistantes et permettre leur ancrage. Une telle disposition présente un intérêt chez la femme, mais s'avère contre-performante chez l'homme dont l'urètre doit être fermement soutenu pour rétablir la continence. De même, la bandelette de US-A-2002/0138025 est tricotée sous forme d'un treillis homogène qui présente ainsi des propriétés mécaniques élastiques isotropes, c'est-à-dire sensiblement identiques dans toutes les directions. Une telle disposition n'est pas souhaitable chez l'homme car les contraintes de traction longitudinale, qui seraient à appliquer à la bandelette pour soutenir suffisamment fermement l'urètre, conduiraient le tricot à se détendre en longueur, en se contractant en largeur, avec un risque réel d'endommager l'urètre par un effet de « fil à couper le beurre ».

Le but de la présente invention est de proposer un ensemble chirurgical de traitement de l'incontinence urinaire chez un homme, qui soit efficace, facile à manipuler, notamment en ce qui concerne sa fixation dans le corps du patient, et qui puisse être mis en place par une voie transobturatrice.

A cet effet, l'invention a pour objet un ensemble chirurgical de traitement de l'incontinence urinaire chez un homme, qui comporte :
- au moins une bandelette allongée de soutien de l'urètre d'un patient, comprenant, au moins dans sa partie longitudinale médiane destinée à s'étendre transversalement à l'aplomb de l'urètre lorsque la bandelette est implantée, un tricot de fils qui forment entre eux des espaces libres, ce tricot comprenant une armature constituée de chaînettes longitudinales et un treillis intermédiaire reliant transversalement ces chaînettes, et
- des moyens de suture adaptés pour être accrochés aux chaînettes du tricot en étant rapportés à travers les espaces libres, de manière à joindre la partie médiane de la bandelette avec des tissus anatomiques sous-urétraux en au moins deux zones ponctuelles d'accrochage alignées suivant une direction sensiblement perpendiculaire à la direction longitudinale de la bandelette.

L'utilisation d'une bandelette allongée permet d'implanter cette dernière jusqu'à proximité de tissus anatomiques sous-urétraux, en particulier à proximité du corps spongieux entourant l'urètre et des corps caverneux disposés de part et d'autre du corps spongieux. De par sa faible largeur, notamment par rapport à des implants de type plaque, les structures anatomiques du patient peuvent en effet être dégagées plus profondément, sans compliquer les gestes du chirurgien. La forme de bandelette allongée permet également d'implanter la bandelette par voie transobturatrice, chaque partie d'extrémité libre de la bandelette pouvant être passée à travers l'un des trous obturateurs de l'os iliaque du patient, ce qui conduit la bandelette à présenter alors une forme globale de U produisant un effet de soutien efficace pour l'urètre située au fond du U. Bien que la voie d'implantation transobturatrice mise en oeuvre par l'ensemble chirurgical selon l'invention puisse, de prime abord, être rapprochée de la voie transobturatrice mise en oeuvre dans le traitement de l'incontinence urinaire chez la femme, il convient de garder à l'esprit que les contraintes liées à l'amarrage d'une telle bandelette chez l'homme sont radicalement opposées à celles chez la femme : chez la femme, la bandelette est implantée en restant souple au niveau du plancher pelvien, l'anatomie du bas-ventre de la femme ne nécessitant et ne supportant aucun ancrage ferme, tel qu'un ancrage par suture. Au contraire, chez l'homme, le corps spongieux entourant l'urètre présente une certaine fermeté et un soutien efficace de l'urètre en vue de traiter l'incontinence nécessite un appui net et sous tension de la bandelette contre ce corps spongieux, ou plus généralement des tissus anatomiques sous-urétraux, ce qui dissuade a priori l'homme du métier de transposer chez l'homme la technique transobturatrice mise au point chez la femme. Une des idées essentielles à la base de l'invention est de mettre à profit la structure tricotée de la bandelette, au moins dans sa partie longitudinale médiane, pour suturer cette partie médiane directement avec les tissus anatomiques sous-urétraux, notamment le corps spongieux. Les moyens de suture utilisés, tels que des fils de suture, traversent aisément les espaces libres formés entre les fils du tricot, sans endommager, par exemple par déchirement, la structure tricotée. L'invention prévoit d'accrocher ces sutures aux chaînettes longitudinales formant l'armature du tricot, en répartissant les zones ponctuelles d'accrochage en un ou plusieurs groupes d'au moins deux zones chacun, alignées perpendiculairement à la direction longitudinale de la bandelette : lors de l'implantation de la bandelette, la traction sur chaque extrémité de la bandelette crée, dans le fond du U formé par la partie longitudinale médiane de la bandelette autour des tissus sous-urétraux, un effet de soutien transversal de l'urètre homogène et bien contrôlé, qui, grâce aux zones d'accrochage suturées, tant à obstruer partiellement l'urètre pour traiter l'incontinence. La présence des chaînettes longitudinales permet au tricot d'être suturé de manière fiable, tout en supportant la traction sans se déformer en largeur. On comprend que la structure tricotée à chaînettes longitudinales de la partie médiane de la bandelette permet à la fois sa suture précise et fiable avec les tissus sous-urétraux et sa forte traction, y compris dans ses zones suturées, nécessaire pour rétablir la continence.

Le fait de suturer la partie médiane de la bandelette permet également d'éviter sa migration ultérieure, notamment vers l'arrière. En outre, les espaces libres formés entre les fils du tricot facilitent la colonisation tissulaire, ce qui explique que les moyens de suture utilisés peuvent être résorbables.

D'autres caractéristiques avantageuses de l'ensemble chirurgical selon l'invention, prises isolément ou suivant toutes les combinaisons techniquement possibles, sont énoncées aux revendications 2 à 10.

L'invention a également pour objet une utilisation d'un ensemble chirurgical tel que défini ci-dessus, pour obtenir un ensemble implanté pour le traitement de l'incontinence chez un homme.

L'invention a en outre pour objet une bandelette allongée de soutien de l'urètre d'un homme pour traiter son incontinence urinaire, comprenant, au moins dans sa partie longitudinale médiane destinée à s'étendre transversalement à l'aplomb de l'urètre lorsque la bandelette est implantée, un tricot de fils qui forment entre eux des espaces libres, ce tricot comprenant une armature constituée de chaînettes longitudinales et un treillis intermédiaire reliant transversalement ces chaînettes, chaînettes auxquelles sont accrochés des moyens de suture en étant rapportés à travers les espaces libres, de manière à joindre la partie médiane de la bandelette avec des tissus anatomiques sous-urètraux en au moins deux zones ponctuelles d'accrochage alignées suivant une direction sensiblement perpendiculaire à la direction longitudinale de la bandelette.

La définition de cette bandelette correspond à la bandelette de l'ensemble chirurgical tel que défini ci-dessus, lorsqu'elle est implantée chez un homme.

L'invention a également pour objet une méthode chirurgicale de traitement de l'incontinence urinaire chez un homme, au moyen d'une bandelette de soutien de l'urètre d'un patient, la bandelette comprenant, au moins dans une partie longitudinale médiane, un tricot de fils qui forment entre eux des espaces libres, ce tricot comprenant une armature constituée de chaînettes longitudinales et un treillis intermédiaire reliant transversalement ces chaînettes, la méthode comprenant des étapes peropératoires consistant à :
- i) inciser verticalement la peau et la graisse sous-cutanée de la région périnéale du patient, entre ses bourses et son anus, jusqu'au bulbe de son corps spongieux urétral, tout en préservant ce corps spongieux ;
- ii) dans l'incision périnéale réalisée lors de l'étape i), dégager, de chaque côté latéral du corps spongieux urétral, les deux corps caverneux du patient ;
- iii) inciser sagitallement l'aponévrose périnéale entre chacun des deux corps caverneux et le corps spongieux urétral ;
- iv) mettre en place la bandelette dans le corps du patient, de manière que la partie médiane de la bandelette s'étend au-dessous et transversalement au corps spongieux urétral tandis que, de chaque côté de cette partie médiane, chaque partie restante de la bandelette s'étend de l'incision correspondante de l'aponévrose périnéale réalisée lors de l'étape iii) jusqu'à la racine de la cuisse correspondante du patient, en passant par le trou obturateur correspondant de l'os iliaque du patient, les extrémités libres de la bandelette s'étendant à l'extérieur du patient depuis respectivement les racines de chaque cuisse ;
- v) tirer sur les deux extrémités libres de la bandelette de manière à mettre le tricot de la partie médiane de la bandelette en appui tendu contre le corps spongieux urétral ;
- vi) rapporter des moyens de suture à travers les espaces libres du tricot et accrocher ces moyens de suture aux chaînettes longitudinales du tricot, de manière à joindre la partie médiane de la bandelette avec des tissus anatomiques sous-urétraux, notamment le corps spongieux urétral, en au moins deux zones ponctuelles d'accrochage alignées suivant une direction sensiblement perpendiculaire à la direction longitudinale de la bandelette ; et
- vii) sectionner les extrémités de la bandelette qui s'étendent à l'extérieur de la racine des cuisses et fermer l'incision périnéale réalisée à l'étape i).

Suivant des caractéristiques avantageuses de cette méthode :
- lors de l'étape vi), les zones ponctuelles d'accrochage aux chaînettes sont réalisées en formant un motif de forme rectangulaire ou carrée, dont une médiatrice est sensiblement perpendiculaire à la direction longitudinale de la bandelette et est située dans le plan sagittal médian de l'urètre ;
- l'étape v) est réalisée avant l'étape vi) ;
- l'étape v) est au moins en partie réalisée après tout ou partie de l'étape vi).

L'invention sera mieux comprise à la lecture de la description qui va suivre, donnée uniquement à titre d'exemple et faite en se référant aux dessins sur lesquels :
- la figure 1 est une vue schématique en perspective d'un ensemble chirurgical selon l'invention ;
- la figure 2 est une vue schématique en élévation du détail cerclé II à la figure 1 ;
- la figure 3 est une section schématique sagittale du bas-ventre d'un homme ;
- la figure 4 est une vue en élévation prise selon la flèche IV de la figure 3, illustrant la mise en oeuvre de l'ensemble de traitement de la figure 1 ; et
- la figure 5 est une section schématique selon la ligne V-V de la figure 4.

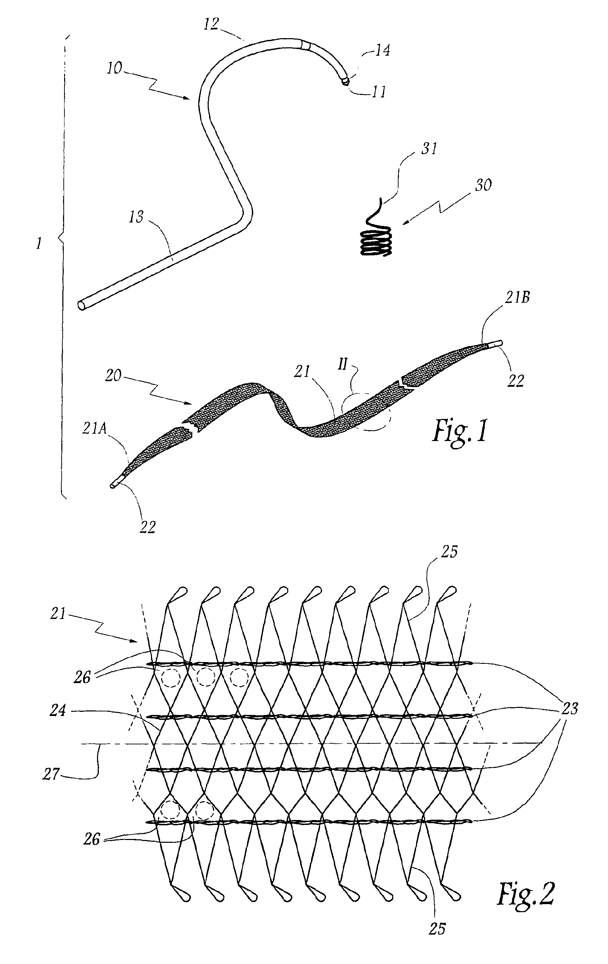

Sur la figure 1 est représenté un ensemble 1 de traitement de l'incontinence urinaire chez un homme. Cet ensemble 1 comprend une aiguille incurvée 10, une bandelette allongée 20 destinée à soutenir l'urètre d'un patient, et une bobine 30 d'un fil de suture 31 résorbable.

L'aiguille 10 est en acier chirurgical et comprend, successivement suivant sa longueur, une extrémité 11 pointue, une partie courante 12 incurvée et une partie de base 13 plus particulièrement affectée à la préhension de l'aiguille. Une poignée facultative, non représentée, peut ainsi être rapportée de manière amovible sur la partie de base 13. L'aiguille 10 utilisée peut présenter des formes de réalisation diverses, notamment en ce qui concerne sa partie incurvée 12 : dans l'exemple représenté à la figure 1, cette partie 12 s'étend globalement dans un seul plan, avec un profil partiellement circulaire dans ce plan, mais d'autres géométries sont aussi envisageables, telles qu'une forme partiellement hélicoïdale.

La bandelette 20 est constituée, sur toute sa longueur, d'un tricot 21 de fils bio-compatibles, dont chaque extrémité longitudinale 21A, 21B est munie d'un moyen 22 de solidarisation à l'aiguille 10. Diverses formes de réalisation sont envisageables pour les moyens de solidarisation 22 : en particulier, chaque moyen 22 se présente sous la forme d'un manchon creux rigide, adapté pour s'encliqueter autour de l'extrémité pointue 11 de l'aiguille 10, dans une rainure circonférentielle 14 ménagée dans le corps de l'aiguille au niveau de cette extrémité. Le lecteur pourra se reporter au document EP-A-1 342 450 au nom de la Demanderesse pour connaître des détails et des variantes de réalisation de ce manchon creux rigide, ainsi que les aménagements correspondants de l'aiguille 10.

Comme représenté plus en détail sur la figure 2, le tricot 21 est constitué de fils, de préférence des monofilaments de polypropylène tricotés selon une structure prédéterminée comprenant essentiellement, d'une part, quatre chaînettes longitudinales tricotées 23, parallèles entre elles et formant l'armature de la structure, et, d'autre part, un treillis intermédiaire maillé 24, reliant transversalement les chaînettes. Cette structure permet au tricot d'être à la fois souple et non extensible, pour permettre sa mise en place dans des tissus mous du patient, en adaptant sa forme à celle de ces tissus, tout en conférant au tricot une élasticité sensiblement nulle suivant sa direction longitudinale, pour permettre d'implanter la bandelette sous forte tension.

La structure du tricot 21 est complétée par deux franges latérales opposées 25, chacune constituée de fils qui s'étendent vers l'extérieur de la bandelette et de manière transversale à la chaînette de bordure 23 correspondante. Ces franges 25 sont destinées à faciliter l'accrochage des tissus anatomiques lorsque la bandelette est implantée dans le corps humain, chacun des fils qui s'étendent depuis l'une ou l'autre des chaînes de bordure 23 présentant avantageusement la forme d'une boucle fermée pour limiter l'irritation des tissus humains lors de la mise en place de la bandelette. Les franges 25 sont ainsi très utiles pour le maintien de la bandelette dans les tissus du patient.

La structure du tricot 21 ménage entre ses fils tricotés des espaces libres importants. En particulier, les espaces libres, référencés 26 à la figure 2, délimités entre chaque chaînette 23 située en bordure du tricot et les maillages du treillis 24 les plus éloignés de la ligne longitudinale centrale 27 du tricot, sont chacun dimensionnés pour pouvoir y inscrire géométriquement un cercle d'au moins 1 mm de diamètre.

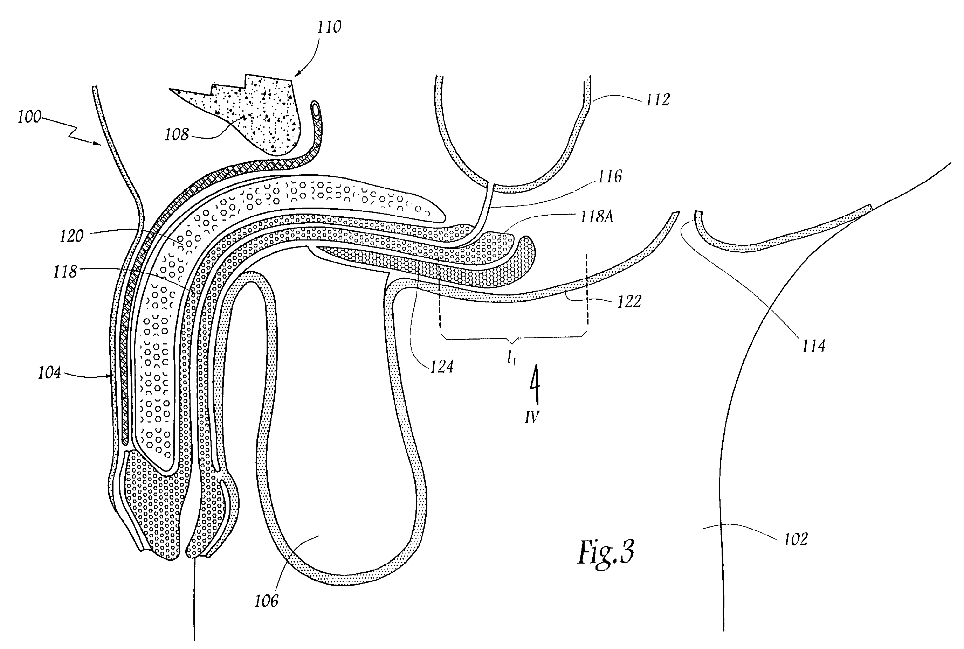

Sur la figure 3, on voit de manière schématique le bas-ventre 100 d'un homme, ainsi que, en arrière-plan, le contour de la partie haute d'une de ses cuisses 102. Dans le plan sagittal correspondant à cette figure, apparaissent successivement, de l'avant vers l'arrière du patient, sa verge 104, le contour d'une de ses bourses 106, le pubis 108 de son os iliaque 110, sa vessie 112 et son anus 114. L'écoulement d'urine depuis la vessie 112 jusqu'à l'extérieur de la verge 104 est réalisé par l'urètre 116 du patient. La majeure partie longitudinale de l'urètre est entourée par le corps spongieux 118 de la verge 104, de part et d'autre duquel s'étendent en longueur les deux corps caverneux 120 de la verge. Sur la figure 3, un seul des corps caverneux est représenté, étant entendu que les deux corps caverneux sont situés de part et d'autre et de manière sensiblement symétrique au plan sagittal du patient, tandis que l'urètre 116 et le corps spongieux 118 s'étendent de manière globalement centrée sur ce plan.

On va maintenant décrire une méthode chirurgicale de traitement de l'incontinence urinaire chez le patient dont le bas-ventre 100 est représenté à la figure 3, au moyen de l'ensemble 1 des figures 1 et 2.

Juste avant l'intervention chirurgicale proprement dite, le patient, sous anesthésie, est sondé par une sonde urétro-vésicale et installé dans une position gynécologique.

Le chirurgien incise verticalement la région périnéale du patient, entre, à l'arrière, l'anus 114 et, à l'avant, les bourses 106, comme indiqué par la référence I₁ sur les figures 3 à 5. La limite avant de l'incision I₁ est donnée par le repérage, avec le doigt, du bord inférieur du pubis 108, tandis que le milieu de l'incision est repéré par le coude que forme l'urètre 116 en direction de la vessie 112, ce coude étant mis en relief par la sonde urétro-vésicale. L'incision I₁ traverse vers le haut à la fois la peau 122 et la graisse sous-cutanée 124 de la région périnéale, et est poursuivie jusqu'à atteindre le bulbe terminale 118A du corps spongieux 118, qui est toutefois préservé. De chaque côté latéral de ce bulbe, on voit alors apparaître les reliefs arrondis blanchâtres de l'albuginée des corps caverneux 120. Par palpation, de chaque côté du bulbe 118A, le chirurgien met alors en évidence le dièdre entre chaque corps caverneux 120 et le corps spongieux 118.

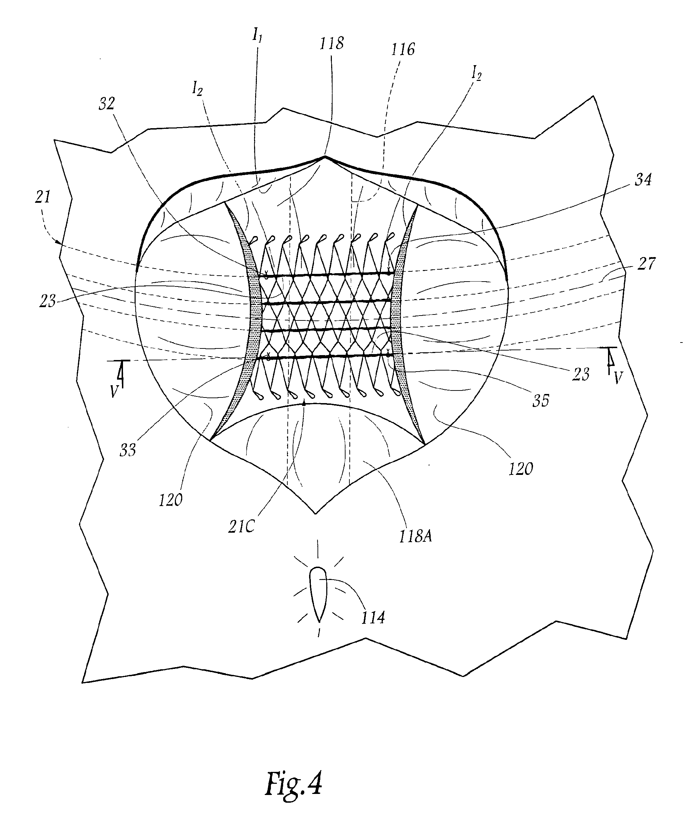

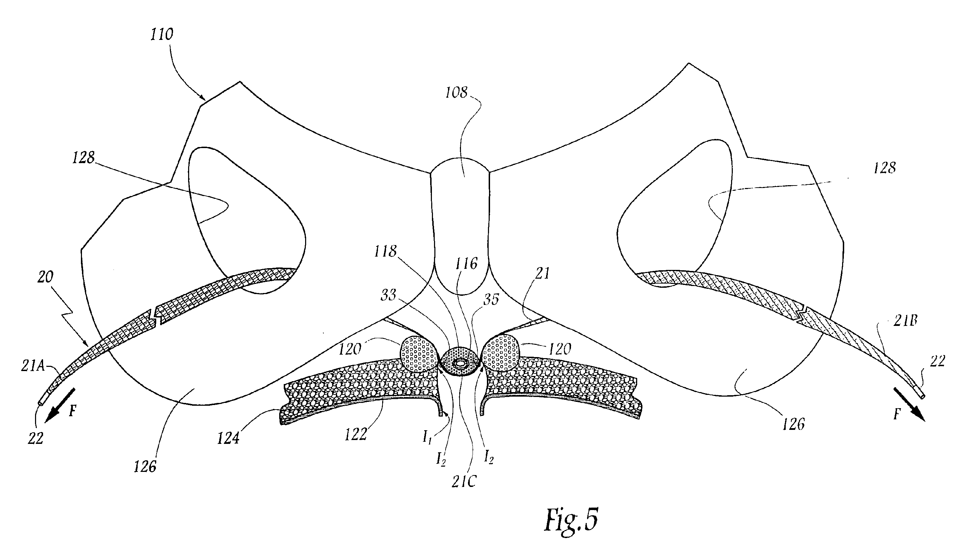

Au fond de chaque dièdre ainsi mis en évidence, l'aponévrose périnéale est incisée de manière sensiblement sagittale, par exemple à l'aide d'un bistouri électrique, sur une profondeur d'environ 1 cm, si possible sans entrer dans le corps caverneux correspondant 120, comme indiqué par les références I₂ sur les figures 4 et 5. Au doigt, le chirurgien palpe et libère doucement les tissus incisés de l'aponévrose, en contournant par dessus chaque corps caverneux 120, pour aller au contact de la hanche ischio-pubienne correspondante 126 de l'os iliaque 110 (figure 5).

Le chirurgien se saisit ensuite de l'aiguille 10 et l'introduit dans le corps du patient, à partir de la racine d'une première de ses cuisses 102. Le point d'entrée de l'extrémité pointue 11 de l'aiguille au niveau de la racine de la cuisse est situé à la fois à environ 4 cm de l'incision I₁ et à environ 4 cm du bord inférieur du relief du muscle grand adducteur de la cuisse. Grâce à sa partie courante incurvée 12, l'aiguille 10 progresse dans les tissus du patient tout en restant du même côté latéral du patient que sa première cuisse 102, jusqu'à atteindre le trou obturateur correspondant 128 de l'os iliaque, à travers lequel l'extrémité pointue 11 progresse jusqu'à rejoindre l'incision I₂ correspondante. Pour ce faire, le chirurgien introduit un doigt dans l'incision I₂, de manière à percevoir l'extrémité pointue 11 en approche et guider ainsi sa sortie dans cette incision.

Une fois que l'extrémité pointue 11 de l'aiguille a franchi l'incision I₂, la bandelette 20 est fixée sur l'aiguille grâce au moyen de solidarisation 22 encliqueté dans la rainure 14, puis l'aiguille est ressortie en la tirant par sa partie de base 13, de sorte que l'extrémité 11 suit la trajectoire inverse l'ayant amenée jusqu'à la région périnéale. La bandelette 20 est ainsi mise en place dans le corps du patient entre une première des deux incisions I₂ et la racine de la première cuisse 102 du patient, en passant par un premier des deux trous obturateurs 128 de l'os iliaque 110. L'aiguille 10 est ainsi tractée vers l'extérieur jusqu'à ce que l'extrémité 21A du tricot 21 s'étende à l'extérieur du patient depuis la racine de sa cuisse. Le chirurgien dégage alors la bandelette de l'aiguille, en déconnectant le moyen de solidarisation 22.

Avec la même aiguille 10 ou une aiguille analogue, le chirurgien réalise des gestes symétriques pour le second côté latéral du patient. L'aiguille est introduite depuis la racine de la seconde cuisse 102, jusqu'à ce que son extrémité pointue 11 rejoigne et s'étende à l'extérieur de la seconde incision I₂ de l'aponévrose entre le second corps caverneux 120 et le corps spongieux 118, en passant par le second trou obturateur 128. Puis l'extrémité 21B de la bandelette est solidarisée à l'extrémité pointue 11, grâce au moyen de solidarisation 22 correspondant. Par traction et retrait de l'aiguille, la bandelette est mise en place dans le corps du patient entre la seconde incision I₂ et la racine de la seconde cuisse 102, en passant par le second trou obturateur 128 et avec l'extrémité 21B du tricot s'étendant à l'extérieur du patient depuis la racine de sa cuisse. La bandelette 20 est alors globalement dans la configuration d'implantation illustrée aux figures 4 et 5, c'est-à-dire avec sa ligne centrale 27 s'étendant de manière globalement perpendiculaire au plan sagittal médian de l'urètre 116.

Le chirurgien met alors la bandelette 20 sous tension, c'est-à-dire, comme indiqué par les flèches F à la figure 5, qu'il tire sur chaque extrémité 21A, 21 B du tricot 21, de manière qu'une partie longitudinale médiane 21C de la bandelette s'appuie de manière franche contre le corps spongieux 118, par le dessous de ce corps. La partie 21C du tricot 21 présente alors une forme de U, au fond duquel le corps spongieux 118 est soutenu. La tension exercée sur la bandelette est réglée de manière à obtenir un soutien obstructif de l'urètre 116, afin de traiter l'incontinence.

Une fois que le chirurgien a sensiblement réglé la tension de la bandelette, cette dernière doit être ancrée dans le patient, pour éviter le relâchement de l'effet sur l'urètre : le chirurgien utilise alors le fil de suture 31 et accroche la partie 21C du tricot 21 au corps spongieux 118 au niveau de quatre zones ponctuelles d'accrochage, référencées respectivement 32 à 35 à la figure 4. Chaque zone d'accrochage est obtenue en introduisant un segment du fil 31 dans l'un des espaces libres 26 et en formant une boucle autour de la chaînette de bordure correspondante 23, une partie du corps spongieux 118 étant coincée à l'intérieur de la boucle. Le passage du fil 31 dans l'un des espaces libres 26 n'endommage pas la structure du tricot 21, le diamètre du fil 31 étant prévu plus petit que les dimensions transversales de ces espaces libres 26. Le diamètre du fil 31 est par exemple de 1 mm.

Comme représenté à la figure 4, les zones d'accrochage 32 et 33 d'une part, et les zones d'accrochage 34 et 35 d'autre part, sont alignées suivant des directions respectives sensiblement perpendiculaires à la ligne centrale 27 du tricot 21. De cette façon, les risques de comportement dissymétrique de la bandelette sont limités, l'avant de la bandelette n'ayant, par exemple, pas tendance à migrer de manière différenciée par rapport à l'arrière de la bandelette. Par ailleurs, les zones d'accrochage 32 à 35 forment un motif en rectangle dont la médiatrice antéropostérieure est sensiblement située dans le plan sagittal médian de l'urètre 116, ce qui favorise un comportement homogène de la bandelette de part et d'autre de l'urètre. De plus, en prévoyant que chaque zone d'accrochage 32 à 35 est plus près de l'un des bords latéraux de la partie de tricot 21C que de la ligne centrale 27, la majeure partie de la largeur du tricot 21 participe à l'effort de soutien obstructif.

Une fois que la bandelette est tendue contre et amarrée au corps spongieux 118, l'intervention chirurgicale prend fin : les extrémités 21A et 21 B extériorisées au niveau des racines des cuisses 102 sont sectionnées, tandis que l'incision périnéale I₁ est refermée par plans, avec si besoin la fermeture préalable de chaque incision I₂.

On note que l'incision verticale périnéale I₁ s'étend de manière sensiblement perpendiculaire à la direction longitudinale de la bandelette 20 une fois que cette dernière est implantée : cette voie d'abord permet une bonne exposition de la zone d'implantation de la bandelette et limite, en postopératoire, la surface de contact entre la bandelette et les plans de fermeture de cette incision.

En variante à la méthode chirurgicale décrite ci-dessus, l'étape de traction des extrémités 21A et 21B de la bandelette 20 et l'étape d'accrochage par suture de la partie médiane 21C du tricot 21 peuvent être inversées ou réalisées de manière concomitante.

Une variante non représentée consiste à implanter simultanément deux bandelettes 20, de manière adjacente l'une à l'autre suivant leur longueur. Le principal intérêt de cette disposition est de doubler la largeur du soutien de l'urètre 116. En pratique, les deux bandelettes implantées doivent pouvoir être tendues contre et amarrées au corps spongieux 118 sans induire un comportement dissymétrique de chaque bandelette par rapport à l'autre. Pour ce faire, les deux bandelettes sont reliées l'une à l'autre au niveau de leur bord respectif adjacent. Une première solution pratique consiste, lors de la fabrication de ces bandelettes, à entremêler les franges adjacentes 25 des bandelettes, ce qui revient alors en fait à disposer d'une « super-bandelette » dont le tricot inclut huit chaînettes longitudinales 23 reliées entre elles par, à la fois, les treillis intermédiaires 24 de chaque bandelette et les deux franges entremêlées. Une autre solution consiste à suturer l'une à l'autre les deux chaînettes de bordure appartenant respectivement aux deux bandelettes. Dans ce cas, avantageusement, cette suture est au moins en partie réalisée au niveau d'une des zones ponctuelles d'accrochage telles que présentées ci-dessus : les deux chaînettes de bordure sont, au niveau de cette ou ces zones, accrochées à la fois l'une à l'autre et au corps spongieux 118, tandis qu'au moins deux autres zones ponctuelles d'accrochage, une antérieure et l'autre postérieure, sont respectivement prévues pour les deux bandelettes, étant entendu que ces au moins trois zones d'accrochage sont alignées suivant une direction sensiblement perpendiculaire à la direction longitudinale des deux bandelettes.

Divers aménagements et variantes à l'ensemble 1 et à la méthode de traitement décrits ci-dessus sont en outre envisageables. A titre d'exemples :
- les chaînettes 23 de la bandelette 20 ne sont pas nécessairement au nombre de quatre de sorte qu'un nombre supérieur ou inférieur est possible, étant entendu qu'au moins deux chaînettes sont indispensables pour obtenir au moins deux zones d'accrochage, respectivement postérieure et antérieure ;
- dans la mesure où les tissus biologiques du patient vont avoir tendance à coloniser la structure du tricot 21, l'utilisation d'un fil de suture 31 résorbable est suffisante ; toutefois, l'utilisation d'un fil de suture non résorbable est envisageable ;
- la structure tricotée illustrée à la figure 2 peut n'être prévue que pour la partie médiane 21C de la bandelette 20, puisque seule cette partie de la bandelette est destinée à s'étendre transversalement à l'aplomb de l'urètre et à être accrochée aux tissus anatomiques sous-urétraux par des segments du fil de suture 31 ; chaque partie d'extrémité libre de la bandelette peut donc en variante présenter une structure différente, étant remarqué que le fait de disposer d'une bandelette constituée sur toute sa longueur du tricot 21 s'avère pratique à fabriquer et facilite le réglage de la tension de la bandelette puisque cette dernière présente alors un comportement bien homogène sur toute sa longueur ;
- d'autres formes de moyens de suture que les fils 31 sont envisageables, telles que des agrafes, des clips, etc., dans la mesure où ces moyens de suture peuvent être accrochés aux fils du tricot 21 sans les endommager, en passant à travers les espaces libres 26 ;
- le nombre des zones d'accrochage suturées 32 à 35 n'est pas nécessairement égal à quatre, bien que cette solution se révèle particulièrement efficace en pratique ; une solution à uniquement deux zones ponctuelles d'accrochage est possible, à condition que ces deux zones soient sensiblement alignées suivant une direction perpendiculaire à la direction longitudinale de la bandelette, pour des raisons de comportement symétrique de l'avant et de l'arrière de la bandelette, les zones d'accrochage étant alors de préférence positionnées sensiblement dans le plan sagittal médian de l'urètre pour homogénéiser le comportement de la bandelette lors de sa traction de part et d'autre de l'urètre ; et/ou
- en plus de suturer la partie médiane de la bandelette 20, il n'est pas exclu de suturer ses extrémités 21A et 21 B, ainsi que ses parties intermédiaires entre sa partie médiane et ses extrémités, étant entendu que l'accrochage de la bandelette est réalisé dans des tissus différents et distants des tissus sous-urètraux accrochés au niveau des zones 32 à 35.

## Revendications

1. Ensemble chirurgical (1) de traitement de l'incontinence urinaire chez un homme, **caractérisé en ce qu'**il comporte :
- au moins une bandelette allongée (20) de soutien de l'urètre (116) d'un patient, comprenant, au moins dans sa partie longitudinale médiane (21C) destinée à s'étendre transversalement à l'aplomb de l'urètre lorsque la bandelette est implantée, un tricot (21) de fils qui forment entre eux des espaces libres (26), ce tricot comprenant une armature constituée de chaînettes longitudinales (23) et un treillis intermédiaire (24) reliant transversalement ces chaînettes, et
- des moyens de suture (31) adaptés pour être accrochés aux chaînettes du tricot en étant rapportés à travers les espaces libres, de manière à joindre la partie médiane de la bandelette avec des tissus anatomiques sous-urétraux (118) en au moins deux zones ponctuelles d'accrochage (32 à 35) alignées suivant une direction sensiblement perpendiculaire à la direction longitudinale (27) de la bandelette.

2. Ensemble suivant la revendication 1, **caractérisé en ce que** chaque espace libre (26) du tricot (21), à travers lequel les moyens de suture (31) sont prévus pour passer, est dimensionné pour pouvoir y inscrire géométriquement un cercle d'au moins 1 mm de diamètre.

3. Ensemble suivant l'une des revendications 1 ou 2, **caractérisé en ce que** le tricot (21) a une structure présentant quatre zones ponctuelles (32 à 35) d'accrochage aux chaînettes (23), formant un motif de forme rectangulaire ou carrée, dont une médiatrice est sensiblement perpendiculaire à la direction longitudinale (27) de la bandelette (20), de telle sorte que cette médiatrice est destinée à être située dans le plan sagittal médian de l'urètre (116) lorsque la bandelette est implantée.

4. Ensemble suivant l'une des revendications 1 ou 2, **caractérisé en ce que** le tricot (21) a une structure présentant uniquement deux zones ponctuelles d'accrochage aux chaînettes (23), de telle sorte que ces deux zones sont destinées à être positionnées dans le plan sagittal médian de l'urètre (116) lorsque la bandelette (20) est implantée.

5. Ensemble suivant l'une quelconque des revendications précédentes, **caractérisé en ce que** le tricot (21) a une structure présentant chaque zone ponctuelle (32 à 35) d'accrochage aux chaînettes (23) plus près d'un des bords latéraux de la bandelette (20) que de sa ligne centrale (27).

6. Ensemble suivant l'une quelconque des revendications précédentes, **caractérisé en ce que** la bandelette (20) est constituée, sur toute sa longueur, du tricot (21).

7. Ensemble suivant l'une quelconque des revendications précédentes, **caractérisé en ce que** les fils du tricot (21) sont des monofilaments de polypropylène.

8. Ensemble suivant l'une quelconque des revendications précédentes, **caractérisé en ce que** les moyens de suture comprennent des fils de suture (31) qui sont individuellement accrochés à l'une des chaînettes (23) du tricot (21), au niveau d'une des zones ponctuelles d'accrochage propre (32 à 35).

9. Ensemble suivant l'une quelconque des revendications précédentes, **caractérisé en ce que** les moyens de suture (31) sont résorbables.

10. Ensemble suivant l'une quelconque des revendications précédentes, **caractérisé en ce que** sont prévues au moins deux bandelettes (20) aptes à être implantées de manière adjacente l'une à l'autre suivant leur longueur, en étant reliées l'une à l'autre au niveau de leur bord respectif adjacent, en particulier en au moins une des zones ponctuelles d'accrochage qui est alors en prise avec une des chaînettes (23) du tricot (21) de chaque bandelette.

11. Utilisation d'un ensemble chirurgical selon l'une quelconque des revendications 1 à 10 pour obtenir un ensemble implanté pour le traitement de l'incontinence urinaire chez un homme.

12. Bandelette allongée (20) de soutien de l'urètre (116) d'un homme pour traiter son incontinence urinaire, comprenant, au moins dans sa partie longitudinale médiane (21 C) s'étendant transversalement à l'aplomb de l'urètre, un tricot (21) de fils qui forment entre eux des espaces libres (26), ce tricot comprenant une armature constituée de chaînettes longitudinales (23) et un treillis intermédiaire (24) reliant transversalement ces chaînettes, chaînettes auxquelles sont accrochés des moyens de suture (31) en étant rapportés à travers les espaces libres, de manière à joindre la partie médiane de la bandelette avec des tissus anatomiques sous-urètraux (118) en au moins deux zones ponctuelles d'accrochage (32 à 35) alignées suivant une direction sensiblement perpendiculaire à la direction longitudinale (27) de la bandelette.
